# EUROPEAN PATENT SPECIFICATION

(11) **EP 1 032 366 B1**
(45) Date of publication and mention of the grant of the patent: **12.02.2003**
(21) Application number: 98951622.4
(22) Date of filing: 13.11.1998
(51) Int. Cl.: A61K 7/50

(54) **PERSONAL CLEANSING WIPE ARTICLES HAVING SUPERIOR SOFTNESS**
KÖRPERREINIGUNGSWISCHGEGENSTÄNDE MIT VERBESSERTER WEICHHEIT
ARTICLES DE TYPE SERVIETTES D'HYGIENE CORPORELLE, DOTES D'UNE DOUCEUR AMELIOREE

(30) Priority: 19.11.1997 US 66207 P
(43) Date of publication of application: 06.09.2000
(73) Proprietor: THE PROCTER & GAMBLE COMPANY, Cincinnati, Ohio 45202 (US)
(72) Inventor: PUNG, David, John, Loveland, Ohio 45140 (US); HEDGES, Steven, Kirk, Fairfield, OH 45014 (US); LINN, Frank, Loveland, OH 45140 (US)
(74) Representative: McKinney, Victoria
(86) International application number: IB9801806
(87) International publication number: WO99025318

(56) References cited:
- EP-A- 0 418 493
- WO-A-96/02701
- WO-A-96/37652
- WO-A-98/18441

## Description

### TECHNICAL FIELD

The present invention relates to personal cleansing wipe articles which have superior softness, feel and cleansing properties. The personal cleansing wipes of the present invention comprise a single layer, nonwoven, hydroentangled substrate and an aqueous liquid cleansing composition coated onto or impregnated into the substrate.

### BACKGROUND OF THE INVENTION

Consumers have in the past used absorbent sheets impregnated with topical compositions for a variety of purposes. For example, wipes impregnated with cleansing compositions are frequently used to conveniently wash hands and face while traveling or in public or anytime when water and soap are not readily accessible. Such wipes are also frequently used on babies to clean up after a bowel movement.

Some consumers have indicated a desire that personal cleansing wipes, baby wipes or other wipes for use on human skin feel softer to the skin than wipes which are currently commercially available. Applicants have now found that softer wipes compositions can be provided by utilizing particular nonwoven, patterned substrates made from hydroentangled fibers in combination with an aqueous cleansing composition.

Substrates which are made from hydroentangled fibers are well known in the art. See, for example, Evans; U.S. Patent 3,485,706; issued December 23, 1969. Patterned substrates are also known in the art. The prior art describes two basis ways of forming a pattern on a nonwoven substrate: mechanical embossing and aperturing. Patterned substrates produced by these prior art methods are associated with consumer-negative. Mechanical embossing involves the application of force to a web through rigid members, such as protrusions on the periphery of a roll, to create areas of high density in the substrate, without changing the basis weight of the high density areas. Unfortunately, the mechanical embossing process can provide a pattern at the expense of other properties desired by the consumer. In particular, embossing can disrupt the bonds between fibers, thereby reducing the tensile strength of the web. Additionally, the pattern may not remain intact when the substrate is wetted. Aperturing involves the creation of a network of fiber bundles around a series of holes or apertures. These patterns are not three-dimensional; raised portions do not exist on the sheet. Although providing a visual effect, this type of pattern does not result in increased softness to the substrate.

It is an object of the present invention to provide wipes articles which are impregnated with an aqueous cleansing composition which have superior softness, feel and cleansing properties compared to prior art compositions.

It is further an object of the present invention to accomplish superior softness, feel and cleansing without incurring consumer-negatives with respect to other aspects of the wipes article.

### SUMMARY OF THE INVENTION

The present invention relates to personal cleansing wipes articles, as defined in claim 1, and to a process for preparing personal cleansing wipe articles, as defined in claim 9. Preferred embodiments are defined in the dependent claims.

### DETAILED DESCRIPTION OF THE INVENTION

The present invention relates to personal cleansing wipes articles which have superior softness, feel and cleansing properties. As used herein, the term "personal cleansing wipes article" refers to products in which a sheet of porous or absorbent material has been impregnated with a personal cleansing composition for the purpose of rubbing the wipe product over a surface (e.g. skin) to clean the surface. The personal cleansing wipes articles of the present invention comprise a particular substrate and a liquid aqueous cleansing composition which is coated onto or impregnated into the substrate. The ingredients used to prepare the personal cleansing wipes articles of the present invention, as well as process for preparing them, are described in detail as follows:

### I. Ingredients

### A. The Substrate

The personal cleansing wipes compositions of the present invention comprise a single layer, nonwoven substrate formed from hydroentangled fibers. By "nonwoven" is meant that the substrate is comprised of fibers which are not woven into a fabric but rather are formed into a sheet, mat, or pad layer having suitable basis weight, caliper, absorbency and strength characteristics.

The fibers from which the substrate is formed can be natural, synthetic, or mixtures thereof. Suitable natural fibers from which to prepare the substrates herein include, for example, wood pulp, wool, silk, jute, hemp, cotton, linen, sisal, ramie and mixtures thereof. Suitable synthetic fibers from which the substrates herein can be prepared include rayon, cellulose ester, polyvinyl derivatives, polyolefins, polyamides, acetate, acrylic, modacrylic fibers, polyester, polyurethane foam, and mixtures thereof. Specific examples of some of these synthetic materials include acrylics such as acrilan, creslan, and the acrylonitrile-based fiber, orlon; cellulose ester fibers such as cellulose acetate, arnel, and acele; polyamides such as nylons (e.g., nylon 6, nylon 66, nylon 610, and the like); polyesters such as fortrel, kodel, and the polyethylene terephthalate fiber, dacron; polyolefins such as polypropylene, polyethylene; polyvinyl acetate fibers; polyurethane foams and mixtures thereof. These and other suitable fibers and the nonwoven materials prepared therefrom are generally described in Riedel, "Nonwoven Bonding Methods and Materials," Nonwoven World (1987); The Encyclopedia Americana, vol. 11, pp. 147-153, and vol. 26, pp. 566-581 (1984); U.S. Patent No. 4,891,227, to Thaman et al., issued January 2, 1990; and U.S. Patent No. 4,891,228.

The substrate employed in the present invention is formed from hydroentangled fibers. One way to prepare a substrate from hydroentangled fibers is to position a web of fibers on a topographical support member comprising an essentially planar background surface with at least one recessed region significantly displaced from the background surface of the forming plate. Typically, the support member comprises a multiplicity of recessed regions, positioned as depressions is some predetermined array, that will form a desired pattern of raised portions on the nonwoven substrate. The fibrous web is presoaked or wetted out with water while on this support member to ensure that as it is treated it will remain on the support member. The support member with the fibrous web thereon is passed under a series of orifices from each of which a fluid, such as water, is ejected under high pressure and directed toward the upper surface of the fibrous web, i.e., that surface of the web which is out of contact with the topographical support member. Initially, these fluid forces "mold" the starting web to the three dimensional support member; as the process of applying fluid force continues, the fibers are entangled and locked together so as to provide a nonwoven substrate comprising a base surface and one or more discrete, raised fibrous regions which are permanently positioned with respect to one another. The water is then transported away from the support member, preferably using a vacuum. The fibrous web is de-watered. The de-watered formed substrate is removed from the support member. The formed substrate is passed over a series of drying drums to dry the substrate. The substrate can then be finished or otherwise processed as desired. Processes for preparing hydroentangled webs are well known in the art. See, for example, Evans; U.S. Patent 3,485,786; issued December 23, 1969; Kalwarres; U.S. Patent 2,862,251 and Griswold; U.S. Patent 3,025,585, all of which describe hydroentangling procedures generally. See also U.S. Patent 5,674,591; James et al; issued October 7, 1997 which specifically describes a hydroentangling process, including the apparatus used in said process, which can be used to prepare the patterned substrates employed in the present invention. U.S. Patent 5,674,591.

The substrates of the present invention are preferably single layer substrates. By "single layer" is meant that the substrate is removed from the topographical support layer as a single, unitary piece, and does not require the addition of other fibrous or nonfibrous structural components. In particular, a scrim is not joined to the nonwoven substrate employed in the present invention. It is understood that the single layer substrates herein can include one or more fiber types, including different synthetic fibers, different natural fibers, and/or a combination of synthetic and natural fibers. Such different fibers can be blended in a homogenous manner through the thickness of the substrate, or alternatively, can be arranged in strata through the thickness of the substrate.

As hereinbefore described, the particular nonwoven, hydroentangled substrates of the present invention comprise a base surface having on a substantial portion of the base surface a three-dimensional pattern comprising a plurality of discrete, raised fibrous regions.

In one embodiment of the present invention, the basis weight and the density, respectively, of the raised fibrous regions are the same as the basis weight and density, respectively, of the base surface. As used herein, the term "basis weight" is the weight of a unit area of fibrous web or portion thereof being characterized. As used herein, the term "density" is the weight of a unit volume of a fibrous web or portion thereof being characterized. Traditional embossing patterning processes produce regions of varying density.

In a second embodiment of the present invention, the basis weight of the raised fibrous portions is greater than the basis weight of the base surface and the density of the raised fibrous regions are the same as the density of the base surface. In a third embodiment of the present invention, the basis weight of some of the raised fibrous regions are substantially the same as the basis weight of the base surface and the basis weight of other raised fibrous regions are greater than the basis weight of the base surface. In this embodiment, as in the other embodiments, the density of all of the raised fibrous areas is essentially the same as the density of the base surface.

The raised fibrous regions of the substrate herein are joined to the base surface of the substrate herein by a fibrous transition region. The fibrous transition region comprises a fiber-poor region and a fiber-rich region. The fibrous transition region, including both the fiber-poor region and fiber-rich region are described in detail in U.S. Patent 5,674,591.

The average basis weight of the nonwoven, patterned substrates used in the present invention ranges from about 40 to about 90 grams per square meter, preferably from about 40 to about 75 grams per square meter, more preferably from about 50 to about 65 grams per square meter as measured by INDA Standard Test IST 130.1. The caliper of the substrates employed in the present invention ranges from about 0.3 to about 1.05 mm, preferably from about 0.5 to about 1.00 mm, more preferably from about 0.6 to about 0.9 mm, as measured by INDA Standard Test IST 120.1 (95).

The substrates of the present invention can be, but are not necessarily, appertured.

### B. The Aqueous Liquid Cleansing Composition

### 1. The Cleansing Surfactant

The personal cleansing wipes articles of the present invention comprise an effective amount of a cleansing surfactant to provide a cleansing benefit. Typically, the aqueous cleansing composition used in the present invention comprises from about 0.5% to about 12.5%, preferably from about 0.75% to about 11%, and more preferably from about 1% to about 10%, and most preferably from about 1% to about 5%, based on the weight of the cleansing composition, of a cleansing surfactant. Preferably, these surfactants or combinations of surfactants should be mild, which means that these surfactants provide sufficient cleansing or detersive benefits but do not overly dry the skin.

A wide variety of cleansing surfactants are useful herein and include those selected from the group consisting of anionic surfactants, nonionic surfactants, amphoteric surfactants, cationic surfactants, and mixtures thereof.

### a. Anionic Surfactants

Nonlimiting examples of anionic surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Patent No. 3,929,678, to Laughlin et al., issued December 30, 1975.

A wide variety of anionic surfactants are useful herein. Nonlimiting examples of anionic surfactants include those selected from the group consisting of sarcosinates, sulfates, isethionates, taurates, phosphates, and mixtures thereof. Amongst the isethionates, the alkoyl isethionates are preferred, and amongst the sulfates, the alkyl and alkyl ether sulfates are preferred. The alkoyl isethionates typically have the formula RCO-OCH₂CH₂SO₃M wherein R is alkyl or alkenyl of from about 10 to about 30 carbon atoms, and M is a water-soluble cation such as ammonium, sodium, potassium and triethanolamine. Nonlimiting examples of these isethionates include those alkoyl isethionates selected from the group consisting of ammonium cocoyl isethionate, sodium cocoyl isethionate, sodium lauroyl isethionate, and mixtures thereof.

The alkyl and alkyl ether sulfates typically have the respective formulae ROSO₃M and RO(C₂H₄O)ₓSO₃M, wherein R is alkyl or alkenyl of from about 10 to about 30 carbon atoms, x is from about 1 to about 10, and M is a water-soluble cation such as ammonium, sodium, potassium and triethanolamine. Another suitable class of anionic surfactants are the water-soluble salts of the organic, sulfuric acid reaction products of the general formula:

R₁--SO₃--M

wherein R₁ is chosen from the group consisting of a straight or branched chain, saturated aliphatic hydrocarbon radical having from about 8 to about 24, preferably about 10 to about 16, carbon atoms; and M is a cation. Still other anionic synthetic surfactants include the class designated as succinamates, olefin sulfonates having about 12 to about 24 carbon atoms, and b-alkyloxy alkane sulfonates. Examples of these materials are sodium lauryl sulfate and ammonium lauryl sulfate.

Other anionic materials include the sarcosinates, nonlimiting examples of which include sodium lauroyl sarcosinate, sodium cocoyl sarcosinate, and ammonium lauroyl sarcosinate.

Other anionic materials useful herein are soaps (i.e. alkali metal salts, e.g., sodium or potassium salts) of fatty acids, typically having from about 8 to about 24 carbon atoms, preferably from about 10 to about 20 carbon atoms. The fatty acids used in making the soaps can be obtained from natural sources such as, for instance, plant or animal-derived glycerides (e.g., palm oil, coconut oil, soybean oil, castor oil, tallow, lard, etc.) The fatty acids can also be synthetically prepared. Soaps are described in more detail in U.S. Patent No. 4,557,853, cited above.

Other anionic materials include phosphates such as monoalkyl, dialkyl, and trialkylphosphate salts.

Other anionic materials include alkanoyl sarcosinates corresponding to the formula RCON(CH₃)CH₂CH₂CO₂M wherein R is alkyl or alkenyl of about 10 to about 20 carbon atoms, and M is a water-soluble cation such as ammonium, sodium, potassium and trialkanolamine (e.g., triethanolamine), a preferred example of which is sodium lauroyl sarcosinate.

Also useful are taurates which are based on taurine, which is also known as 2-aminoethanesulfonic acid. Examples of taurates include N-alkyltaurines such as the one prepared by reacting dodecylamine with sodium isethionate according to the teaching of U.S. Patent 2,658,072.

Nonlimiting examples of preferred anionic surfactants useful herein include those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl sarcosinate, and mixtures thereof.

Especially preferred for use herein are ammonium lauryl sulfate and ammonium laureth sulfate.

### b. Nonionic Surfactants

Nonlimiting examples of nonionic surfactants for use in the compositions of the present invention are disclosed in McCutcheon's, Detergents and Emulsifiers, North American edition (1986), published by allured Publishing Corporation; and McCutcheon's, Functional Materials, North American Edition (1992).

Nonionic surfactants useful herein include those selected from the group consisting of polyoxyethylenes, alkyl glucosides, alkyl polyglucosides, polyhydroxy fatty acid amides, alkoxylated fatty acid esters, sucrose esters, amine oxides, and mixtures thereof.

Alkyl glucosides and alkyl polyglucosides are useful herein, and can be broadly defined as condensation products of long chain alcohols, e.g. C8-30 alcohols, with sugars or starches or sugar or starch polymers, i.e., glycosides or polyglycosides. These compounds can be represented by the formula (S)ₙ-O-R wherein S is a sugar moiety such as glucose, fructose, mannose, and galactose; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a C8-20 alkyl group, and n is an integer of from about 1 to about 9. Commercially available examples of these surfactants include decyl polyglucoside (available as APG 325 CS from Henkel) and lauryl polyglucoside (available as APG 600CS and 625 CS from Henkel). Also useful are sucrose ester surfactants such as sucrose cocoate and sucrose laurate.

Other useful nonionic surfactants include polyhydroxy fatty acid amide surfactants, more specific examples of which include glucosamides, corresponding to the structural formula: 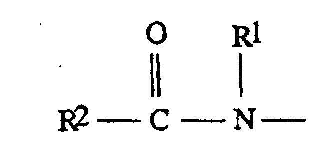 wherein: R¹ is H, C₁-C₄ alkyl, 2-hydroxyethyl, 2-hydroxy- propyl, preferably C₁-C₄ alkyl, more preferably methyl or ethyl, most preferably methyl; R² is C₅-C₃₁ alkyl or alkenyl, preferably C₇-C₁₉ alkyl or alkenyl, more preferably C₉-C₁₇ alkyl or alkenyl, most preferably C₁₁-C₁₅ alkyl or alkenyl; and Z is a polhydroxyhydrocarbyl moiety having a linear hydrocarbyl chain with a least 3 hydroxyls directly connected to the chain, or an alkoxylated derivative (preferably ethoxylated or propoxylated) thereof. Z preferably is a sugar moiety selected from the group consisting of glucose, fructose, maltose, lactose, galactose, mannose, xylose, and mixtures thereof. An especially preferred surfactant corresponding to the above structure is coconut alkyl N-methyl glucoside amide (i.e., wherein the R²CO- moiety is derived from coconut oil fatty acids). Processes for making compositions containing polyhydroxy fatty acid amides are disclosed, for example, in G.B. Patent Specification 809,060, published February 18, 1959, by Thomas Hedley & Co., Ltd.; U.S. Patent No. 2,965,576, to E.R. Wilson, issued December 20, 1960; U.S. Patent No. 2,703,798, to A.M. Schwartz, issued March 8, 1955; and U.S. Patent No. 1,985,424, to Piggott, issued December 25, 1934.

Other examples of nonionic surfactants include amine oxides. Amine oxides correspond to the general formula R₁R₂R₃NO, wherein R₁ contains an alkyl, alkenyl or monohydroxy alkyl radical of from about 8 to about 18 carbon atoms, from 0 to about 10 ethylene oxide moieties, and from 0 to about 1 glyceryl moiety, and R₂ and R₃ contain from about 1 to about 3 carbon atoms and from 0 to about 1 hydroxy group, e.g., methyl, ethyl, propyl, hydroxyethyl, or hydroxypropyl radicals. The arrow in the formula is a conventional representation of a semipolar bond. Examples of amine oxides suitable for use in this invention include dimethyl-dodecylamine oxide, oleyldi(2-hydroxyethyl) amine oxide, dimethyloctylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

Nonlimiting examples of preferred nonionic surfactants for use herein are those selected form the group consisting of polyoxyethylenes, C8-C14 glucose amides, C8-C14 alkyl polyglucosides, sucrose cocoate, sucrose laurate, lauramine oxide, cocoamine oxide, and mixtures thereof.

### c. Amphoteric Surfactants

The term "amphoteric surfactant," as used herein, is also intended to encompass zwitterionic surfactants, which are well known to formulators skilled in the art as a subset of amphoteric surfactants.

A wide variety of amphoteric surfactants can be used in the compositions of the present invention. Particularly useful are those which are broadly described as derivatives of aliphatic secondary and tertiary amines, preferably wherein the nitrogen is in a cationic state, in which the aliphatic radicals can be straight or branched chain and wherein one of the radicals contains an ionizable water solubilizing group, e.g., carboxy, sulfonate, sulfate, phosphate, or phosphonate.

Nonlimiting examples of amphoteric surfactants useful in the compositions of the present invention are disclosed in McCutcheon's, Detergents and Emulsifiers, North American edition (1986), published by allured Publishing Corporation; and McCutcheon's, Functional Materials, North American Edition (1992).

Nonlimiting examples of amphoteric or zwitterionic surfactants are those selected from the group consisting of betaines, sultaines, hydroxysultaines, alkyliminoacetates, iminodialkanoates, aminoalkanoates, and mixtures thereof.

Examples of betaines include the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine (available as Lonzaine 16SP from Lonza Corp.); lauryl bis-(2-hydroxyethyl) carboxymethyl betaine, oleyl dimethyl gamma-carboxypropyl betaine, lauryl bis-(2-hydroxypropyl)alpha-carboxyethyl betaine, coco dimethyl sulfopropyl betaine, lauryl dimethyl sulfoethyl betaine, lauryl bis-(2-hydroxyethyl) sulfopropyl betaine, amidobetaines and amidosulfobetaines (wherein the RCONH(CH₂)₃ radical is attached to the nitrogen atom of the betaine), oleyl betaine (available as amphoteric Velvetex OLB-50 from Henkel), and cocamidopropyl betaine (available as Velvetex BK-35 and BA-35 from Henkel).

Examples of sultaines and hydroxysultaines include materials such as cocamidopropyl hydroxysultaine (available as Mirataine CBS from Rhone-Poulenc).

Preferred for use herein are amphoteric surfactants having the following structure: 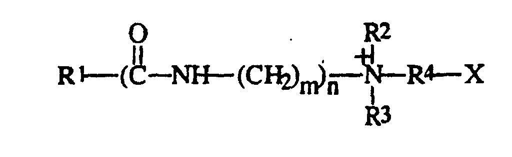 wherein R¹ is unsubstituted, saturated or unsaturated, straight or branched chain alkyl having from about 9 to about 22 carbon atoms. Preferred R¹ has from about 11 to about 18 carbon atoms; more preferably from about 12 to about 18 carbon atoms; more preferably still from about 14 to about 18 carbon atoms; m is an integer from 1 to about 3, more preferably from about 2 to about 3, and more preferably about 3; n is either 0 or 1, preferably 1; R² and R³ are independently selected from the group consisting of alkyl having from 1 to about 3 carbon atoms, unsubstituted or mono-substituted with hydroxy, preferred R² and R³ are CH₃; X is selected from the group consisting of CO₂, SO₃ and SO₄; R⁴ is selected from the group consisting of saturated or unsaturated, straight or branched chain alkyl, unsubstituted or monosubstituted with hydroxy, having from 1 to about 5 carbon atoms. When X is CO₂, R⁴ preferably has 1 or 3 carbon atoms, more preferably 1 carbon atom. When X is SO₃ or SO₄, R⁴ preferably has from about 2 to about 4 carbon atoms, more preferably 3 carbon atoms.

Examples of amphoteric surfactants of the present invention include the following compounds:
Cetyl dimethyl betaine (this material also has the CTFA designation cetyl betaine) 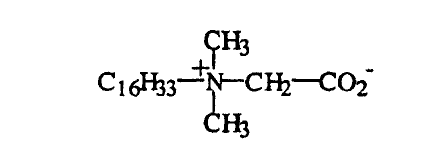
Cocamidopropylbetaine 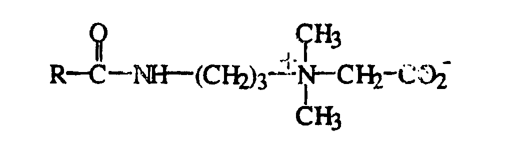 wherein R has from about 9 to about 13 carbon atoms
Cocamidopropyl hydroxy sultaine 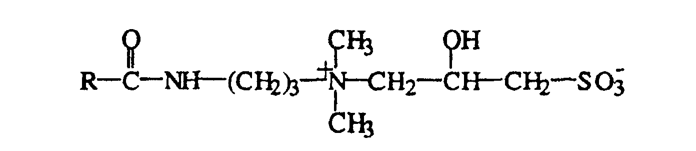
wherein R has from about 9 to about 13 carbon atoms,

Examples of other useful amphoteric surfactants are alkyliminoacetates, and iminodialkanoates and aminoalkanoates of the formulas RN[CH₂)ₘCO₂M]₂ and RNH(CH₂)ₘCO₂M wherein m is from 1 to 4, R is a C₈-C₂₂ alkyl or alkenyl, and M is H, alkali metal, alkaline earth metal ammonium, or alkanolammonium. Also included are imidazolinium and ammonium derivatives. Specific examples of suitable amphoteric surfactants include sodium 3-dodecyl-aminopropionate, sodium 3-dodecylaminopropane sulfonate, N-higher alkyl aspartic acids such as those produced according to the teaching of U.S. Patent 2,438,091; and the products sold under the trade name "Miranol" and described in U.S. Patent 2,528,378. Other examples of useful amphoterics include amphoteric phosphates, such as coamidopropyl PG-dimonium chloride phosphate (commercially available as Monaquat PTC, from Mona Corp.). Also useful are amphoacetates such as disodium lauroamphodiacetate, sodium lauroamphoacetate, and mixtures thereof.

### d. Cationic Surfactants

Examples of cationic surfactants useful herein are disclosed in McCutcheon's, Detergents and Emulsifiers, North American edition (1986). published by allured Publishing Corporation; and McCutcheon's, Functional Materials, North American Edition (1992).

Examples of cationic surfactants useful herein include cationic alkyl ammonium salts such as those having the formula:

R₁ R₂ R₃ R₄ N⁺ X⁻

wherein R₁, is selected from an alkyl group having from about 12 to about 18 carbon atoms, or aromatic, aryl or alkaryl groups having from about 12 to about 18 carbon atoms; R₂, R₃, and R₄ are independently selected from hydrogen, an alkyl group having from about 1 to about 18 carbon atoms, or aromatic, aryl or alkaryl groups having from about 12 to about 18 carbon atoms; and X is an anion selected from chloride, bromide, iodide, acetate, phosphate, nitrate, sulfate, methyl sulfate, ethyl sulfate, tosylate, lactate, citrate, glycolate, and mixtures thereof. Additionally, the alkyl groups can also contain ether linkages, or hydroxy or amino group substituents (e.g., the alkyl groups can contain polyethylene glycol and polypropylene glycol moieties).

More preferably, R₁ is an alkyl group having from about 12 to about 18 carbon atoms; R₂ is selected from H or an alkyl group having from about 1 to about 18 carbon atoms; R₃ and R₄ are independently selected from H or an alkyl group having from about 1 to about 3 carbon atoms; and X is as described in the previous paragraph.

Most preferably, R₁ is an alkyl group having from about 12 to about 18 carbon atoms; R₂, R₃, and R₄ are selected from H or an alkyl group having from about 1 to about 3 carbon atoms; and X is as described previously.

Alternatively, other useful cationic surfactants include amino-amides, wherein in the above structure R₁ is alternatively R₅CO-(CH₂)ₙ -, wherein R₅ is an alkyl group having from about 12 to about 22 carbon atoms, and n is an integer from about 2 to about 6, more preferably from about 2 to about 4, and most preferably from about 2 to about 3. Nonlimiting examples of these cationic emulsifiers include stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

Nonlimiting examples of quaternary ammonium salt cationic surfactants include those selected from the group consisting of cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide, lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl dimethyl ammonium chloride, distearyl methyl ammonium bromide, and mixtures thereof. Additional quaternary ammonium salts include those wherein the C12 to C22 alkyl carbon chain is derived from a tallow fatty acid or from a coconut fatty acid. The term "tallow" refers to an alkyl group derived from tallow fatty acids (usually hydrogenated tallow fatty acids), which generally have mixtures of alkyl chains in the C16 to C18 range. The term "coconut" refers to an alkyl group derived from a coconut fatty acid, which generally have mixtures of alkyl chains in the C12 to C14 range. Examples of quaternary ammonium salts derived from these tallow and coconut sources include ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chloride, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

Preferred cationic surfactants useful herein include those selected from the group consisting of dilauryl dimethyl ammonium chloride, distearyl dimethyl ammonium chloride, dimyristyl dimethyl ammonium chloride, dipalmityl dimethyl ammonium chloride, distearyl dimethyl ammonium chloride, and mixtures thereof.

### 2. Optional Ingredients

### a. Conditioning Ingredients

The aqueous cleansing composition used in the personal cleansing wipes of the present invention can optionally and preferably comprise an lipophilic skin conditioning agent which is useful for providing a conditioning benefit to the skin during the use of the product. The aqueous cleansing solution employed in the present invention typically comprises from about 0.1% to about 30%, preferably from about 0.5% to about 20%, preferably from about 1% to about 10%, and more preferably from about 1% to about 5% by weight of the aqueous cleansing composition.

The lipophilic skin conditioning agent is selected from one or more oil soluble conditioning agents such that the weighted arithmetic mean solubility parameter of the lipophilic skin conditioning agent is less than or equal to 10.5. It is recognized, based on this mathematical definition of solubility parameters, that it is possible, for example, to achieve the required weighted arithmetic mean solubility parameter, i.e. less than or equal to 10.5, for alipophilic skin conditioning agent comprising two or more compounds if one of the compounds has an individual solubility parameter greater than 10.5.

Solubility parameters are well known to the formulation chemist of ordinary skill in the art and are routinely used as a guide for determining compatibility's and solubilities of materials in the formulation process.

The solubility parameter of a chemical compound, δ, is defined as the square root of the cohesive energy density for that compound. Typically, a solubility parameter for a compound is calculated from tabulated values of the additive group contributions for the heat of vaporization and molar volume of the components of that compound, using the following equation: 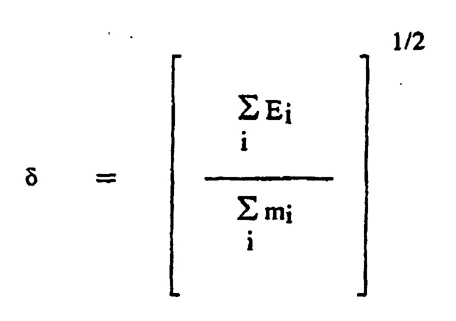 wherein Σᵢ Eᵢ = the sum of the heat of vaporization additive group contributions, and
Σᵢ mᵢ = the sum of the molar volume additive group contributions
Standard tabulations of heat of vaporization and molar volume additive group contributions for a wide variety of atoms and groups of atoms are collected in Barton, A.F.M. Handbook of Solubility Parameters, CRC Press, Chapter 6, Table 3, pp. 64-66 (1985). The above solubility parameter equation is described in Fedors, R.F., "A Method for Estimating Both the Solubility Parameters and Molar Volumes of Liquids", Polymer Engineering and Science, vol. 14, no. 2, pp. 147-154 (February 1974).

Solubility parameters obey the law of mixtures such that the solubility parameter for a mixture of materials is given by the weighted arithmetic mean (i.e. the weighted average) of the solubility parameters for each component of that mixture. See, Handbook of Chemistry and Physics, 57th edition, CRC Press, p. C-726 (1976-1977).

Formulation chemists typically report and use solubility parameters in units of (cal/cm³)^{1/2}. The tabulated values of additive group contributions for heat of vaporization in the Handbook of Solubility Parameters are reported in units of kJ/mol. However, these tabulated heat of vaporization values are readily converted to cal/mol using the following well-known relationships:
1 J/mol = 0.239006 cal/mol and 1000 J = 1 kJ.
See Gordon, A.J. et al., The Chemist's Companion, John Wiley & Sons, pp. 456-463, (1972).

Solubility parameters have also been tabulated for a wide variety of chemical materials. Tabulations of solubility parameters are found in the above-cited Handbook of Solubility Parameters. Also, see "Solubility Effects In Product, Package, Penetration, And Preservation", C.D. Vaughan, Cosmetics and Toiletries, vol. 103, October 1988, pp. 47-69.

Nonlimiting examples of lipophilic skin conditioning agents useful herein include those selected from the group consisting of mineral oil, petrolatum, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkarylsiloxanes, cylcomethicones having 3 to 9 silicon atoms, vegetable oils, hydrogenated vegetable oils, polypropylene glycol C4-C20 alkyl ethers, di C8-C30 alkyl ethers, and mixtures thereof.

Mineral oil, which is also known as petrolatum liquid, is a mixture of liquid hydrocarbons obtained from petroleum. See The Merck Index, Tenth Edition, Entry 7048, p. 1033 (1983) and International Cosmetic Ingredient Dictionary, Fifth Edition, vol. 1, p.415-417 (1993).

Petrolatum, which is also known as petroleum jelly, is a colloidal system of nonstraight-chain solid hydrocarbons and high-boiling liquid hydrocarbons, in which most of the liquid hydrocarbons are held inside the micelles. See The Merck Index, Tenth Edition, Entry 7047, p. 1033 (1983); Schindler, Drug, Cosmet. Ind., **89,** 36-37, 76, 78-80, 82 (1961); and International Cosmetic Ingredient Dictionary, Fifth Edition, vol. 1, p. 537 (1993).

Straight and branched chain hydrocarbons having from about 7 to about 40 carbon atoms are useful herein. Nonlimiting examples of these hydrocarbon materials include dodecane, isododecane, squalane, cholesterol, hydrogenated polyisobutylene, docosane (i.e. a C₂₂ hydrocarbon), hexadecane, isohexadecane (a commercially available hydrocarbon sold as Permethyl® 101A by Presperse, South Plainfield, NJ). Also useful are the C7-C40 isoparaffins, which are C7-C40 branched hydrocarbons.

Also useful are C1-C30 alcohol esters of C1-C30 carboxylic acids and of C2-C30 dicarboxylic acids, including straight and branched chain materials as well as aromatic derivatives. Also useful are esters such as monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, and propylene glycol diesters of C1-C30 carboxylic acids. Straight chain, branched chain and aryl carboxylic acids are included herein. Also useful are propoxylated and ethoxylated derivatives of these materials. Nonlimiting examples include diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenrate, dioctyl maleate, dioctyl sebacate, diisopropyl adipate, cetyl octanoate, diisopropyl dilinoleate, caprilic/capric triglyceride, PEG-6 caprylic/capric triglyceride, PEG-8 caprylic/capric triglyceride, and mixtures thereof.

Also useful are various C1-C30 monoesters and polyesters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3:4 molar ratio. A preferred solid material is sucrose polyester in which the degree of esterification is 7-8, and in which the fatty acid moieties are C18 mono- and/or di-unsaturated and behenic, in a molar ratio of unsaturates:behenic of 1:7 to 3:5. A particularly preferred solid sugar polyester is the octaester of sucrose in which there are about 7 behenic fatty acid moieties and about 1 oleic acid moiety in the molecule. Other materials include cottonseed oil or soybean oil fatty acid esters of sucrose. The ester materials are further described in, U.S. Patent No. 2,831,854, U.S. Patent No. 4,005,196, to Jandacek, issued January 25, 1977; U.S. Patent No. 4,005,195, to Jandacek, issued January 25, 1977, U.S. Patent No. 5,306,516, to Letton et al., issued April 26, 1994; U.S. Patent No. 5,306,515, to Letton et al., issued April 26, 1994; U.S. Patent No. 5,305,514, to Letton et al., issued April 26, 1994; U.S. Patent No. 4,797,300, to Jandacek et al., issued January 10, 1989; U.S. Patent No. 3,963,699, to Rizzi et al, issued June 15, 1976; U.S. Patent No. 4,518,772, to Volpenhein, issued May 21, 1985; and U.S. Patent No. 4,517,360, to Volpenhein, issued May 21, 1985.

Nonvolatile silicones such as polydialkylsiloxanes, polydiarylsiloxanes, and polyalkarylsiloxanes are also useful oils. These silicones are disclosed in U.S. Patent No. 5,069,897, to Orr, issued December 3, 1991. The polyalkylsiloxanes correspond to the general chemical formula R₃SiO[R₂SiO]ₓSiR₃ wherein R is an alkyl group (preferably R is methyl or ethyl, more preferably methyl) and x is an integer up to about 500, chosen to achieve the desired molecular weight. Commercially available polyalkylsiloxanes include the polydimethylsiloxanes, which are also known as dimethicones, nonlimiting examples of which include the Vicasil® series sold by General Electric Company and the Dow Corning® 200 series sold by Dow Corning Corporation. Specific examples of polydimethylsiloxanes useful herein include Dow Corning® 225 fluid having a viscosity of 10 centistokes and a boiling point greater than 200°C, and Dow Corning® 200 fluids having viscosities of 50, 350, and 12,500 centistokes, respectively, and boiling points greater than 200°C. Also useful are materials such as trimethylsiloxysilicate, which is a polymeric material corresponding to the general chemical formula [(CH₂)₃SiO_{1/2}]ₓ[SiO₂]_{y}, wherein x is an integer from about 1 to about 500 and y is an integer from about 1 to about 500. A commercially available trimethylsiloxysilicate is sold as a mixture with dimethicone as Dow Corning® 593 fluid. Also useful herein are dimethiconols, which are hydroxy terminated dimethyl silicones. These materials can be represented by the general chemical formulas R₃SiO[R₂SiO]ₓSiR₂OH and HOR₂SiO[R₂SiO]ₓSiR₂OH wherein R is an alkyl group (preferably R is methyl or ethyl, more preferably methyl) and x is an integer up to about 500, chosen to achieve the desired molecular weight. Commercially available dimethiconols are typically sold as mixtures with dimethicone or cyclomethicone (e.g. Dow Corning ® 1401, 1402, and 1403 fluids). Also useful herein are polyalkylaryl siloxanes, with polymethylphenyl siloxanes having viscosities from about 15 to about 65 centistokes at 25°C being preferred. These materials are available, for example, as SF 1075 methylphenyl fluid (sold by General Electric Company) and 556 Cosmetic Grade phenyl trimethicone fluid (sold by Dow Corning Corporation).

Vegetable oils and hydrogenated vegetable oils are also useful herein. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, and mixtures thereof.

Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl ether, PPG-15 stearyl ether,, dioctyl ether, dodecyl octyl ether, and mixtures thereof.

### b. Active Ingredients

The personal cleansing wipes of the present invention can also optionally comprise a safe and effective amount of one or more active ingredients or pharmaceutically-acceptable salts thereof.

The term "safe and effective amount" as used herein, means an amount of an active ingredient high enough to modify the condition to be treated or to deliver the desired skin benefit, but low enough to avoid serious side effects, at a reasonable benefit to risk ratio within the scope of sound medical judgment. What is a safe and effective amount of the active ingredient will vary with the specific active, the ability of the active to penetrate through the skin, the age, health condition, and skin condition of the user, and other like factors.

The active ingredients useful herein can be categorized by their therapeutic benefit or their postulated mode of action. However, it is to be understood that the active ingredients useful herein can in some instances provide more than one therapeutic benefit or operate via more than one mode of action. Therefore, classifications herein are made for the sake of convenience and are not intended to limit the active ingredient to that particular application or applications listed. Also, pharmaceutically-acceptable salts of these active ingredients are useful herein. The following active ingredients are useful in the compositions of the present invention.
Anti-Acne Actives: Examples of useful anti-acne actives include the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.
Anti-Wrinkle and Anti-Skin Atrophy Actives: Examples of antiwrinkle and anti-skin atrophy actives include retinoic acid and its derivatives (e.g., cis and trans); retinol; retinyl esters; niacinamide, salicylic acid and derivatives thereof; sulfur-containing D and L amino acids and their derivatives and salts, particularly the N-acetyl derivatives, a preferred example of which is N-acetyl-L-cysteine; thiols, e.g. ethane thiol; hydroxy acids, phytic acid, lipoic acid; lysophosphatidic acid, and skin peel agents (e.g., phenol and the like).
Non-Steroidal Anti-Inflammatory Actives (NSAIDS): Examples of NSAIDS include the following categories: propionic acid derivatives; acetic acid derivatives; fenamic acid derivatives; biphenylcarboxylic acid derivatives; and oxicams. All of these NSAIDS are fully described in U.S. Patent 4,985,459 to Sunshine et al., issued January 15, 1991. Examples of useful NSAIDS include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Also useful are the steroidal anti-inflammatory drugs including hydrocortisone and the like.
Topical Anesthetics: Examples of topical anesthetic drugs include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.
Artificial Tanning Agents and Accelerators. Examples of artificial tanning agents and accelerators include dihydroxyacetaone, tyrosine, tyrosine esters such as ethyl tyrosinate, and phospho-DOPA.
Antimicrobial and Antifungal Actives: Examples of antimicrobial and antifungal actives include β-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa xylenol, nystatin, tolnaftate, zinc pyrithione and clotrimazole.

Preferred examples of actives useful herein include those selected from the group consisting of salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, phytic acid, N-acetyl-L-cysteine, lipoic acid, azelaic acid, arachidonic acid, benzoylperoxide, tetracycline, ibuprofen, naproxen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, neocycin sulfate, and mixtures thereof.
Sunscreen Actives: Also useful herein are sunscreening actives. A wide variety of sunscreening agents are described in U.S. Patent No. 5,087,445, to Haffey et al., issued February 11, 1992; U.S. Patent No. 5,073,372, to Turner et al., issued December 17, 1991; U.S. Patent No. 5,073,371, to Turner et al. issued December 17, 1991; and Segarin, et al., at Chapter VIII, pages 189 et seq., of Cosmetics Science and Technology. Nonlimiting examples of sunscreens which are useful in the compositions of the present invention are those selected from the group consisting of 2-ethylhexyl *p*-methoxycinnamate, 2-ethylhexyl N,N-dimethyl*-p*-aminobenzoate, *p*-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof. Still other useful sunscreens are those disclosed in U.S. Patent No. 4,937,370, to Sabatelli, issued June 26, 1990; and U.S. Patent No. 4,999,186, to Sabatelli et al., issued March 12, 1991. Especially preferred examples of these sunscreens include those selected from the group consisting of 4-N,N-(2-ethylhexyl)methylaminobenzoic acid ester of 2,4-dihydroxybenzophenone, 4-N,N-(2-ethylhexyl)methylaminobenzoic acid ester with 4-hydroxydibenzoylmethane, 4-N,N- (2-ethylhexyl)-methylaminobenzoic acid ester of 2-hydroxy-4-(2-hydroxyethoxy)benzophenone, 4-N,N-(2-ethylhexyl)-methylaminobenzoic acid ester of 4-(2-hydroxyethoxy)dibenzoylmethane, and mixtures thereof. Exact amounts of sunscreens which can be employed will vary depending upon the sunscreen chosen and the desired Sun Protection Factor (SPF) to be achieved. SPF is a commonly used measure of photoprotection of a sunscreen against erythema. See Federal Register, Vol. 43, No. 166, pp. 38206-38269, August 25, 1978.

Nonlimiting examples of preferred actives useful herein include those selected from the group consisting of salicylic acid, benzoyl peroxide, niacinamide, cis-retinoic acid, trans-retinoic acid, retinol, retinyl palmitate, phytic acid, N-acetyl L-cysteine, azelaic acid, lipoic acid, resorcinol, lactic acid, glycolic acid, ibuprofen, naproxen, hydrocortisone, phenoxyethanol, phenoxypropanol, phenoxyisopropanol, 2,4,4,'-trichloro-2'-hydroxy diphenyl ether, 3,4,4'-trichlorocarbanilide, 2-ethylhexyl *p*-methoxycinnamic acid, oxybenzone, 2-phenylbenzimidozole-5-sulfonic acid, dihydroxyacetone, and mixtures thereof.

### c. Proton Donating Agents

The aqueous cleansing composition comprising the personal cleansing wipes of the present invention can optionally comprise from about 0.1% to about 10%, preferably from about 0.5% to about 8%, more preferably from about 1% to about 5 %, based on the weight of the cleansing composition, of a proton donating agent. By "proton donating agent" it is meant any acid compound or mixture thereof, which results in undissociated acid on the skin after use. Proton donating agents can be organic acids, including polymeric acids, mineral acids or mixtures thereof.

A non-exclusive list of examples of organic acids which can be used as the proton donating agent are adipic acid, tartaric acid, citric acid, maleic acid, malic acid, succinic acid, glycolic acid, glutaric acid, benzoic acid, malonic acid, salicylic acid, gluconic acid, polymeric acids, their salts, and mixtures thereof. A non-exclusive list of examples of mineral acid for use herein are hydrochloric, phosphoric, sulfuric and mixtures thereof.

Polymeric acids are especially preferred acids for use herein from the standpoint that they cause less stinging to the skin than other acids. As used herein, the term "polymeric acid" refers to an acid with repeating units of carboxylic acid groups joined together into one chain. Suitable polymeric acids can include homopolymers, copolymers and terpolymers, but must contain at least 30 mole% carboxylic acid groups. Specific examples of suitable polymeric acids useful herein include straight-chain poly(acrylic) acid and its copolymers, both ionic and nonionic, (e.g., maleic-acrylic, sulfonic-acrylic, and styrene-acrylic copolymers), those cross-linked polyacrylic acids having a molecular weight of less than about 250,000, preferably less than about 100,000 poly (α-hydroxy) acids, poly (methacrylic) acid, and naturally occurring polymeric acids such as carageenic acid, carboxy methyl cellulose, and alginic acid. Straight-chain poly(acrylic) acids are especially preferred for use herein.

In one preferred embodiment of the present invention, the proton donating agent is used to buffer the pH of the aqueous cleansing composition to a pH ranging from about 3.0 to about 6.0, more preferably from about 3.0 to about 5.0 and most preferably from about 3.5 to about 4.5.

### d. Water Soluble Conditioning Agents

The present invention can also optionally comprise water soluble conditioning agents. Such water soluble conditioning agents are typically included in the aqueous cleansing composition employed herein t a level ranging from about 0.1% to about 2%, preferably from about 0.2% to about 1.5%, more preferably from about 0.5% to about 1% by weight of the aqueous cleansing composition.

Nonlimiting examples of conditioning agents useful as water soluble conditioning agents include those selected from the group consisting of polyhydric alcohols, polypropylene glycols, polyethylene glycols, ureas, pyrolidone carboxylic acids, ethoxylated and/or propoxylated C3-C6 diols and triols, alpha-hydroxy C2-C6 carboxylic acids, ethoxylated and/or propoxylated sugars, polyacrylic acid copolymers, sugars having up to about 12 carbons atoms, sugar alcohols having up to about 12 carbon atoms, and mixtures thereof. Specific examples of useful water soluble conditioning agents include materials such as urea; guanidine; glycolic acid and glycolate salts (e.g. ammonium and quaternary alkyl ammonium); lactic acid and lactate salts (e.g. ammonium and quaternary alkyl ammonium); sucrose, fructose, glucose, eruthrose, erythritol, sorbitol, mannitol, glycerol, hexanetriol, propylene glycol, butylene glycol, hexylene glycol, and the like; polyethylene glycols such as PEG-2, PEG-3, PEG-30, PEG-50, polypropylene glycols such as PPG-9, PPG-12, PPG-15, PPG-17, PPG-20, PPG-26, PPG-30, PPG-34; alkoxylated glucose; hyaluronic acid; and mixtures thereof. Also useful are materials such as aloe vera in any of its variety of forms (e.g., aloe vera gel), chitin, starch-grafted sodium polyacrylates such as Sanwet (RTM) IM-1000, IM-1500, and IM-2500 (available from Celanese Superabsorbent Materials, Portsmouth, VA); lactamide monoethanolamine; acetamide monoethanolamine; and mixtures thereof. Also useful are propoxylated glycerols as described in propoxylated glycerols described in U.S. Patent No. 4,976,953, to Orr et al., issued December 11, 1990.

### e. Drying Agents

Drying agents can be employed in the aqueous cleansing compositions employed herein to boost the drying rate of the liquid composition once it applied to the skin via rubbing the wipe product on the skin.. Some aqueous cleansing compositions can give a sticky impression when they are applied to the skin, especially during the time it takes for the composition to dry. It has been found that fast-drying compositions provide a softer, smoother skin feel, with less stickiness. Suitable drying agents include isoparaffin, alcohols and combinations thereof. A mixture of isoparaffin and ethanol is especially preferred. Drying agents are typically included in the cleansing compositions employed herein at a level ranging from about 1% to about 60%, preferably from about 3% to about 40%, more preferably from about 5% to about 20% by weight.

### f. Other Optional Ingredients

The compositions of the present invention can comprise a wide range of other optional components. These additional components should be pharmaceutically acceptable. The CTFA Cosmetic Ingredient Handbook, Second Edition, 1992, which is incorporated by reference herein in its entirety, describes a wide variety of nonlimiting cosmetic and pharmaceutical ingredients commonly used in the skin care industry, which are suitable for use in the compositions of the present invention. Nonlimiting examples of functional classes of ingredients are described at page 537 of this reference. Examples of these and other functional classes include: abrasives, absorbents, anticaking agents, antioxidants, vitamins, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, preservatives, propellants, reducing agents, skin bleaching agents, and sunscreening agents.

Also useful herein are aesthetic components such as fragrances, pigments, colorings, essential oils, skin sensates, astringents, skin soothing agents, and skin healing agents.

### g. Viscosity of Aqueous Cleansing Composition

The aqueous cleansing compositions employed in the wipes product herein preferably have a viscosity in the range of from 0.01 to 10, preferably from 0.05 to 2.0 g cm⁻¹s⁻¹ (1 to about 1000, preferably from 5 to 200 centipoise) as measured by a Brookfield Digital Viscometer, Model DV-II+ Version 3.2 according to the operating instructions set forth in Manual No. M/92-161-H895.

Examples of suitable aqueous cleansing compositions for use herein are set forth in the following U.S. Patents and U.S. Patent Applications: U.S. Patent Application Serial Number 08/727,807 pages 3-6 and 8); U.S. Patent Application Serial Number 08/868,668 filed on June 4, 1997; U.S. Patent 4,941,995 issued July 17, 1990 to Richards et al; U.S. Patent 4,904,524 issued February 27, 1990 to Yoh; U.S. Patent 5,043,155 to Puchalski; U.S. Patent 5,534,265 to Fowler; U.S. Patent 5,648,083 to Blieszner et al.; and U.S. Patent Application Serial Number 08/736,471 filed October 24, 1996.

### II. Preparation of Absorbent Sheets Impregnated with Aqueous Liquid

### Cleansing Solution

Any method suitable for the application of aqueous or aqueous/alcoholic impregnates, including flood coating, spray coating or metered dosing, can be used to impregnate the substrates herein with the emulsion composition described herein. More specialized techniques, such as Meyer Rod, floating knife or doctor blade, which are typically used to impregnate cleansing solutions into absorbent sheets may also be used.

The cleansing solution should preferably comprise from about 100% to about 400%, preferably from about 200% to about 400% by weight of the absorbent sheet.

Prior to or after impregnation of the liquid cleansing solution into the substrate, the substrate may be folded into stacks. The substrate is then typically packaged in any of the moisture and vapor impermeable packages known in the art.

### EXAMPLES

The following are examples of the personal cleansing wipes compositions of the present invention.

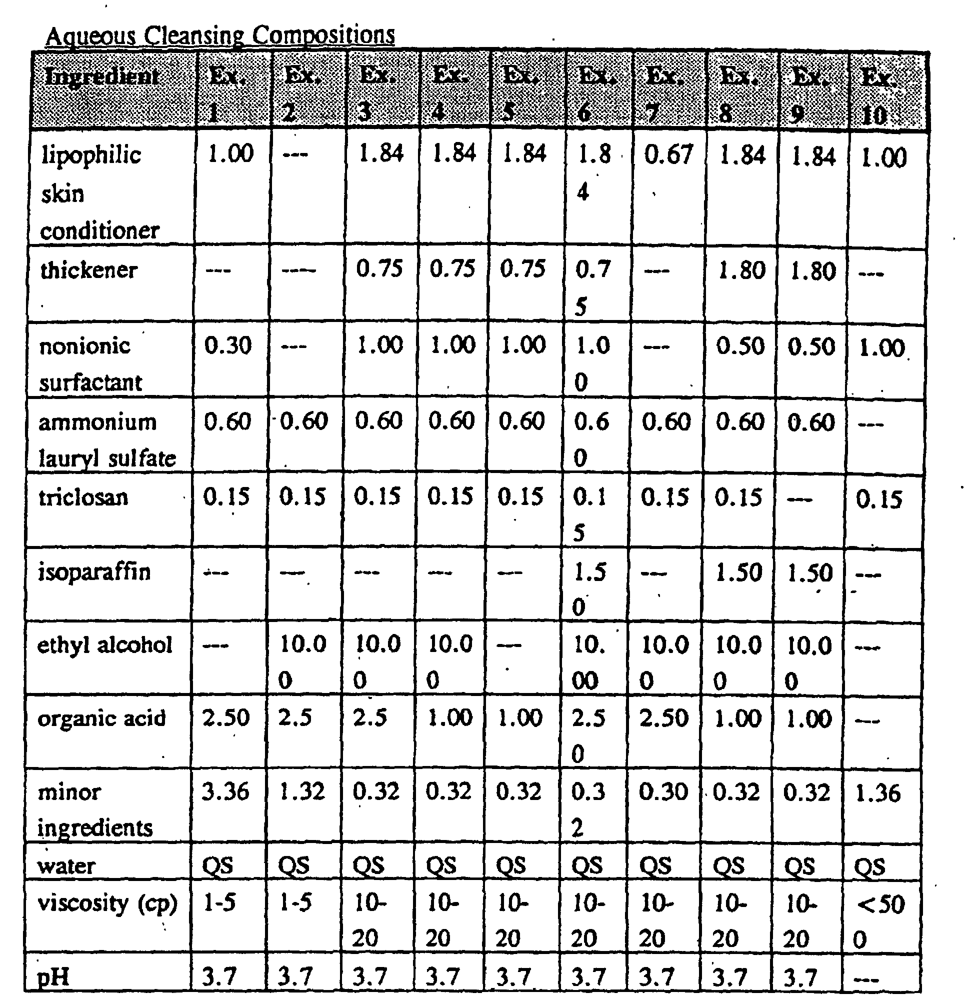

The aqueous cleansing compositions (Examples 1-10) are each impregnated onto substrates of the type described in Examples 1 and 2 of U.S. Patent 5,674, 591 wherein the basis weight of the substrate is 60 grams per square meter and wherein the pattern is a starburst. 230% by weight of the substrate of the aqueous cleansing composition is impregnated into the substrate by pouring the compositions onto the substrate via a cup. Each of the resulting wipes articles will exhibit desirable softness, feel and cleansing properties.

The aqueous cleansing compositions (Examples 1-10) are each impregnated onto substrates of the type described in Examples 1 and 2 of U.S. Patent 5,674, 591 wherein the basis weight of the substrate is 60 grams per square meter and wherein the pattern is a zigzag. 230% by weight of the substrate of the aqueous cleansing composition is impregnated into the substrate by pouring the compositions onto the substrate via a cup. Each of the resulting wipes articles will exhibit desirable softness, feel and cleansing properties.

## Claims

1. A personal cleansing wipe article having superior softness, feel and cleansing properties, which wipe article comprises:
A. a single layer, nonwoven substrate formed from hydroentangled fibers, said substrate having on a substantial portion of a base surface thereof a three-dimensional pattern, which pattern comprises a plurality of discrete, raised fibrous regions, wherein the raised fibrous regions have a density which is the same as the density of the base surface, and wherein said raised fibrous regions are joined to said base surface by a fibrous transition region.; and
B an aqueous liquid cleansing composition comprising an effective amount of a cleansing surfactant, said aqueous liquid cleansing composition being coated onto or impregnated into said substrate to the extent of from 100% to 400% by weight of the substrate.

2. A personal cleansing wipe article according to Claim 1 wherein said raised fibrous regions have a basis weight which is the same as or greater than the basis weight of the base surface.

3. A personal cleansing wipe article according to any one of the preceding Claims, wherein the basis weight of some of the raised fibrous regions is the same as the basis weight of the base surface and wherein the basis weight of the remainder of the raised fibrous regions is greater than the basis weight of the base surface.

4. A personal cleansing wipe article according to any one of the preceding Claims, which has an average basis weight ranging from 40 to 90 grams per square meter and a caliper ranging from 0.3 to 1.05 millimeters.

5. A personal cleansing wipe article according to any one of the preceding Claims, wherein the aqueous cleansing composition comprises:
a. from 0.5% to 12.5% of a surfactant; and
b. from 0.5% to 5% of alipophilic skin moisturizing agent.

6. A personal cleansing wipe article according to any one of the preceding Claims, which additionally comprises from 1% to 60% of a drying agent.

7. A personal cleansing wipe article according to any one of the preceding Claims, wherein the aqueous cleansing composition comprises:
a. from 0.5% to 12.5% of a surfactant;
b. from 0.1% to 10% of an acid; and
c. from 0.5% to 5% of a lipophilic skin moisturizing agent;
and wherein said aqueous cleansing composition has a pH ranging from 3 to 6.

8. A personal cleansing wipe article having superior softness, feel and cleansing properties, which wipe article comprises:
A. a single layer, nonwoven substrate formed from hydroentangled fibers, said substrate having on a substantial portion of a base surface thereof a three-dimensional pattern, which pattern comprises a plurality of discrete, raised fibrous regions, wherein said raised fibrous regions are joined to said base surface by a fibrous transition region.; and
B an aqueous liquid cleansing composition comprising an effective amount of a cleansing surfactant, said aqueous liquid cleansing composition being coated onto or impregnated into said substrate to the extent of from 100% to 400% by weight of the substrate;
wherein said three-dimensional pattern is formed as the fibers are being entangled.

9. A process for preparing a personal cleansing wipe article having superior softness, feel and cleansing properties, which process comprises:
A. placing a web of fibers on a foraminous forming plate or topographical support member comprising an essentially planar background surface with at least one recessed region significantly displaced from the background surface of the forming plate;
B. applying fluid force to the upper surface of the fibrous web such that the fibers become entangled and a patterned substrate is formed;
C. transporting the fluid away from the patterned substrate; and
D. coating or impregnating the patterned substrate with an aqueous cleansing composition comprising an effective amount of a cleansing surfactant to the extent of from 100% to 400% by weight of the substrate.

## Revendications

1. Lingette d'hygiène corporelle présentant des propriétés supérieures de nettoyage, de toucher et de douceur, laquelle lingette comprend :
A. un substrat non tissé monocouche formé de fibres hydro-enchevêtrées, ledit substrat ayant sur une partie sensible de sa surface de base un motif tridimensionnel, lequel motif comprend une pluralité de régions fibreuses relevées discrètes où les régions fibreuses relevées ont une densité qui est la même que celle de la surface de base et où lesdites régions fibreuses relevées sont réunies à ladite surface de base par une région de. transition fibreuse ; et
B. une composition de nettoyage liquide aqueuse comprenant une quantité efficace d'un tensioactif de nettoyage, ladite composition de nettoyage liquide aqueuse étant revêtue ou imprégnée dans ledit substrat à raison de 100 % à 400 % en poids du substrat.

2. Lingette d'hygiène corporelle selon la revendication 1, dans laquelle lesdites régions fibreuses relevées ont un grammage qui est le même ou supérieur au grammage de la surface de base.

3. Lingette d'hygiène corporelle selon l'une quelconque des revendications précédentes, dans laquelle le grammage de certaines des régions fibreuses relevées est le même que celui de la surface de base et dans laquelle le grammage du reste des régions fibreuses relevées est supérieur à celui de la surface de base.

4. Lingette d'hygiène corporelle selon l'une quelconque des revendications précédentes qui a un grammage moyen compris dans la gamme de 40 à 90 g/m² et une épaisseur comprise dans la gamme de 0,3 à 1,05 mm.

5. Lingette d'hygiène corporelle selon l'une quelconque des revendications précédentes, dans laquelle la composition de nettoyage aqueuse comprend:
a. de 0,5 % à 12,5 % d'un tensioactif ; et
b. de 0,5 % à 5 % d'un agent humectant la peau lipophile.

6. Lingette d'hygiène corporelle selon l'une quelconque des revendications précédentes qui comprend, en outre, de 1 % à 60 % d'un agent séchant.

7. Lingette d'hygiène corporelle selon l'une quelconque des revendications précédentes, dans laquelle la composition de nettoyage aqueuse comprend :
a. de 0,5 % à 12,5 % d'un tensioactif ;
b. de 0,1 % à 10 % d'un acide ; et
c. de 0,5 % à 5 % d'un agent humectant la peau lipophile ;
et dans laquelle ladite composition de nettoyage aqueuse a un pH dans la gamme de 3 à 6.

8. Lingette d'hygiène corporelle présentant des propriétés supérieures de nettoyage, de toucher et de douceur, laquelle lingette comprend :
A. un substrat non tissé monocouche formé de fibres hydro-enchevêtrées, ledit substrat ayant sur une partie sensible de sa surface de base un motif tridimensionnel, lequel motif comprend une pluralité de régions fibreuses relevées discrètes où les régions fibreuses relevées sont réunies à ladite surface de base par une région de transition fibreuse ; et
B. une composition de nettoyage liquide aqueuse comprenant une quantité efficace d'un tensioactif de nettoyage, ladite composition de nettoyage liquide aqueuse étant revêtue ou imprégnée dans ledit substrat à raison de 100 % à 400 % en poids du substrat, dans laquelle ledit motif tridimensionnel est formé à mesure que les fibres sont enchevêtrées.

9. Procédé d'obtention de lingette d'hygiène corporelle présentant des propriétés supérieures de nettoyage, de toucher et de douceur, lequel procédé comprend les étapes consistant à :
A. placer une nappe de fibres sur une plaque de formation perforée ou un élément de support topographique comprenant une surface d'arrière-plan essentiellement plane avec au moins une région évidée déplacée de façon significative de la surface d'arrière-plan de la plaque de formation ;
B. appliquer une force fluidique à la surface supérieure de la nappe fibreuse si bien que les fibres deviennent enchevêtrées et qu'un substrat à motif est formé ;
C. transporter le fluide à distance du substrat à motif ; et
D. revêtir ou imprégner le substrat à motif avec une composition de nettoyage aqueuse comprenant une quantité efficace d'un tensioactif de nettoyage à raison de 100 % à 400 % en poids du substrat.

## Patentansprüche

1. Reinigungswischartikel für den persönlichen Gebrauch mit ausgezeichneter Weichheit, Anfühlung und Reinigungseigenschaften, wobei der Wischartikel umfaßt:
A. ein Einzelschicht-Nonwovensubstrat, gebildet aus hydroverknäuelten Fasern, wobei das Susbtrat auf einem wesentlichen Bereich einer Grundfläche hiervon ein dreidimensionales Muster aufweist, wobei das Muster eine Vielzahl diskreter, erhobener, faserförmiger Regionen umfaßt, worin die erhobenen faserförmigen Regionen eine Dichte aufweisen, welche die gleiche ist wie die Dichte der Grundfläche, und wobei die erhobenen faserförmigen Regionen mit der Grundfläche durch eine faserförmige Übergangsregion verbunden sind; und
B. eine wäßrige flüssige Reinigungszusammensetzung, umfassend eine wirksame Menge eines Reinigungstensids, wobei die wäßrige flüssige Reinigungszusammensetzung auf das Substrat beschichtet oder in dieses imprägniert ist bis zu einem Ausmaß von 100 bis 400 Gew.-% des Substrats.

2. Reinigungswischartikel für den persönlichen Gebrauch nach Anspruch 1, wobei die erhobenen faserförmigen Regionen ein Grundgewicht aufweisen, welches gleich ist oder größer als das Grundgewicht der Grundfläche.

3. Reinigungswischartikel für den persönlichen Gebrauch nach mindestens einem der vorangehenden Ansprüche, wobei das Grundgewicht einiger der erhobenen faserförmigen Regionen das gleiche ist wie das Grundgewicht der Grundfläche, und wobei das Grundgewicht der verbleibenden erhobenen faserförmigen Regionen größer ist als das Grundgewicht der Grundfläche.

4. Reinigungswischartikel für den persönlichen Gebrauch nach mindestens einem der vorangehenden Ansprüche, das ein durchschnittliches Grundgewicht im Bereich von 40 bis 90 g pro Quadratmeter und eine Dicke im Bereich von 0,3 bis 1,05 mm aufweist.

5. Reinigungswischartikel für den persönlichen Gebrauch nach mindestens einem der vorangehenden Ansprüche, wobei die wäßrige Reinigungszusammensetzung umfaßt:
a. 0,5% bis 12,5% eines Tensids; und
b. 0,5% bis 5% eines lipophilen Hautfeuchthaltemittels.

6. Reinigungswischartikel für den persönlichen Gebrauch nach mindestens einem der vorangehenden Ansprüche, welcher zusätzlich 1% bis 60% eines Trocknungsmittels umfaßt.

7. Reinigungswischartikel für den persönlichen Gebrauch nach mindestens einem der vorangehenden Ansprüche, wobei die wäßrige Reinigungszusammensetzung umfaßt:
a. 0,5% bis 12,5% eines Tensids;
b. 0,1% bis 10% einer Säure; und
c. 0,5% bis 5% eines lipophilen Hautfeuchthaltemittels;
und wobei die wäßrige Reinigungszusammensetzung einen pH im Bereich von 3 bis 6 ausweist.

8. Reinigungswischartikel für den persönlichen Gebrauch mit ausgezeichneter Weichheit, Anfühlung und Reinigungseigenschaften, wobei der Wischartikel umfaßt:
A. ein Einzelschicht-Nonwovensubstrat, gebildet aus hydroverknäuelten Fasern, wobei das Susbtrat auf einem wesentlichen Teil einer Grundfläche hiervon ein dreidimensionales Muster aufweist, welches Muster eine Vielzahl diskreter, erhobener, faserförmiger Regionen umfaßt, worin die erhobenen, faserförmigen Regionen an die Grundfläche durch eine faserförmige Übergangsregion gebunden sind: und
B. eine wäßrige flüssige Reinigungszusammensetzung, umfassend eine wirksame Menge eines Reinigungstensids, wobei die wäßrige flüssige Reinigungszusammensetzung auf das Substrat beschichtet oder in dieses imprägniert ist bis zu einem Ausmaß von 100 bis 400 Gew.-% des Substrats;
wobei das dreidimensionale Muster gebildet wird, während die Fasern verknäuelt werden.

9. Verfahren zur Herstellung eines Reinigungswischartikels für den persönlichen Gebrauch mit ausgezeichneter Weichheit, Anfühlung und Reinigungseigenschaften, welches Verfahren umfaßt:
A. Platzieren einer Bahn aus Fasern auf einer löchrigen Formgebungsplatte oder topographischem Trägerelement, umfassend eine im wesentlichen planare Hintergrundoberfläche mit mindestens einer ausgesparten Region, welche signifikant von der Hintergrundoberfläche der Formgebungsplatte verlagert ist;
B. Aufbringen einer Fluidkraft auf die obere Oberfläche der faserförmigen Bahn, so dass die Fasern verknäuelt werden und ein Muster aufweisendes Substrat gebildet wird;
C. Transportieren des Fluids weg von dem Muster aufweisenden Substrat; und
D. Beschichten oder Imprägnieren des Muster aufweisenden Substrats mit einer wäßrigen Reinigungszusammensetzung, umfassend eine wirksame Menge eines Reinigungstensids bis zu einem Ausmaß von 100 bis 400 Gew.-% des Substrats.
